(19) Europäisches Patentamt
European Patent Office
Office européen des brevets

(11) **EP 4 570 212 A1**

(12) **EUROPEAN PATENT APPLICATION**

(43) Date of publication:
**18.06.2025 Bulletin 2025/25**

(21) Application number: **24220033.5**

(22) Date of filing: **16.12.2024**

(51) International Patent Classification (IPC):
**A61B 90/00** (2016.01)        **A61B 5/1495** (2006.01)
**A61B 5/00** (2006.01)         **A61B 18/00** (2006.01)

(52) Cooperative Patent Classification (CPC):
**A61B 90/06; A61B 5/1495; A61B 5/6852;**
A61B 5/6885; A61B 2018/00267; A61B 2090/064;
A61B 2090/065; A61B 2560/0223

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **17.12.2023 US 202318542682**

(71) Applicant: **Biosense Webster (Israel) Ltd.
2066717 Yokneam (IL)**

(72) Inventors:
• **GLINER, Vadim**
  **2066717 Yokneam (IL)**
• **SITNITSKY, Ilya**
  **2066717 Yokneam (IL)**
• **HAAS, Eran**
  **2066717 Yokneam (IL)**

(74) Representative: **Carpmaels & Ransford LLP
One Southampton Row
London WC1B 5HA (GB)**

(54) **FORCE AND TORQUE JIG**

(57)     Apparatus for calibrating a catheter, consisting of a jig having a base defining a first axis orthogonal to the base. A frame is coupled to the base, and is configured to rotate about a second axis orthogonal to the first axis. A catheter support is coupled to the frame and is configured to retain the catheter and to rotate the catheter about a third axis orthogonal to the second axis. The jig also has a gauge that is coupled to the base and to a distal end assembly of the catheter. The gauge is configured to provide an indication of a force on the distal end assembly in response to translation of the distal end assembly along the first axis.

FIG. 2

EP 4 570 212 A1

## Description

### FIELD OF THE DISCLOSURE

This disclosure relates generally to catheterization, and specifically to calibrating a catheter.

### BACKGROUND

During a cardiac ablation procedure, contact force between an electrode and tissue being ablated is an important parameter for both pulse-field ablation (PFA) and radiofrequency (RF) ablation. The quality and the depth of the ablation achieved is related to the force applied.

### BRIEF DESCRIPTION OF THE DRAWINGS

The present disclosure will be understood from the following detailed description, taken in conjunction with the drawings in which:

Fig. 1 is a schematic block diagram of a calibration system, according to an example of the present disclosure;

Fig. 2 has schematic perspective views of the calibration system, according to an example of the present disclosure;

Figs. 3A and 3B are schematic figures of a catheter distal end assembly used in the calibration system, according to an example of the present disclosure; and

Fig. 4 is a schematic figure illustrating the relation between axes of the calibration system, according to an example of the present disclosure.

### DESCRIPTION OF EXAMPLES

OVERVIEW

Pulse-field ablation (PFA) and radiofrequency (RF) ablation are cardiac procedures, using a catheter, that may be used to restore a heart to its sinus rhythm. During the procedure a physician manipulates the catheter so that electrodes on the catheter distal end contact selected sites within the heart, and when contact is achieved, the ablation may be activated.

In order to reduce the time for the procedure and simplify the procedure, it is advantageous to use a catheter having a distal end assembly with multiple electrodes, such as a catheter with a basket or a balloon distal end, herein termed a basket-type catheter, so that multiple sites may be contacted simultaneously. In addition, to further reduce the time, in some examples at least some of the electrodes may be activated simultaneously.

The distal end assembly of a basket-type catheter may be inserted into the heart by a minimally invasive method, such as insertion through a femoral artery. The physician manipulates the proximal end of the catheter shaft to position the assembly at a desired location and to engage the electrodes against the tissue with a desired force. While the electrodes are engaged with the tissue, manipulation of the proximal end may exert a torque on the distal end assembly. Since the distal end assembly is flexible, the torque may lead to twisting of the distal end assembly. The inventors have observed that in some cases the applied torque may be followed by the distal end assembly untwisting relatively suddenly, possibly causing trauma to the heart tissue and/or interfering with performance of an ablation.

The distal end assembly includes three-dimensional (3D) position sensors that enable the orientation of the assembly to be measured with respect to the catheter shaft to which the assembly is attached.

An example of the present disclosure provides a jig which is able to measure positions of the 3D sensors of the assembly, so as to measure the twist of the assembly. The jig is able to apply a force and a torque to the distal end assembly, and in addition is able to measure the force and the torque applied.

The data acquired from the jig enables a correspondence to be formed between the orientation of the assembly with respect to the catheter shaft and the force and torque applied to the distal end assembly. During a procedure this correspondence may be used to find the force and the torque on a basket-type catheter distal end assembly used in the procedure. Knowledge of the value of the torque enables a warning to be issued if the torque exceeds a preset limit, so as to prevent sudden untwisting of the catheter distal end assembly during the procedure.

SYSTEM DESCRIPTION

In the following description, like elements are identified by the same numeral, and are differentiated, where required, by having a letter attached as a suffix to the numeral.

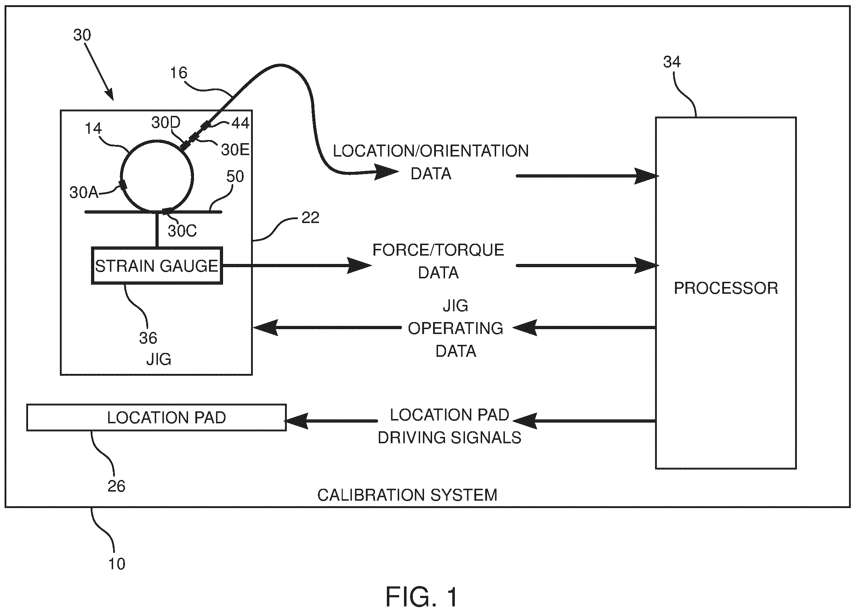

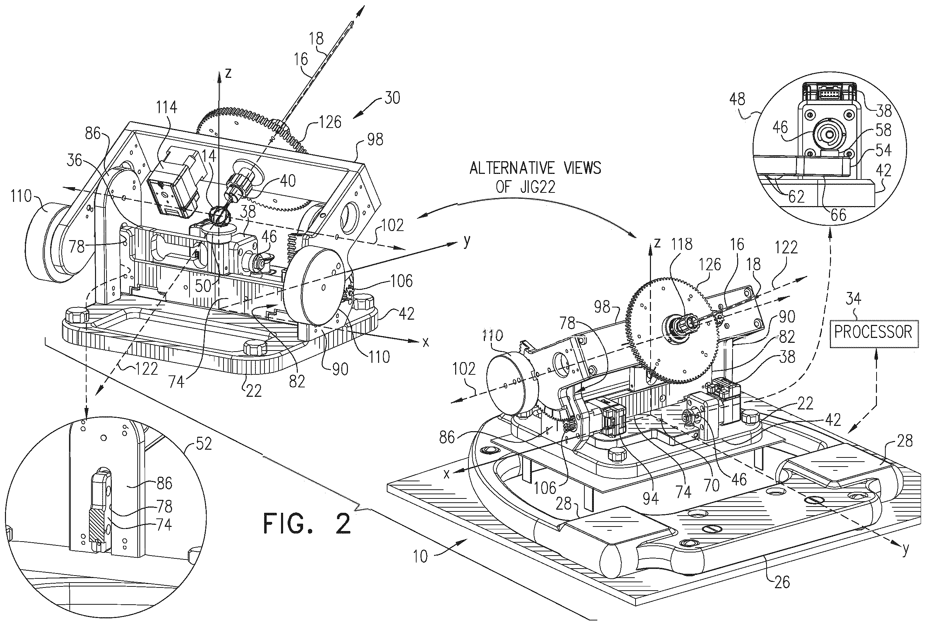

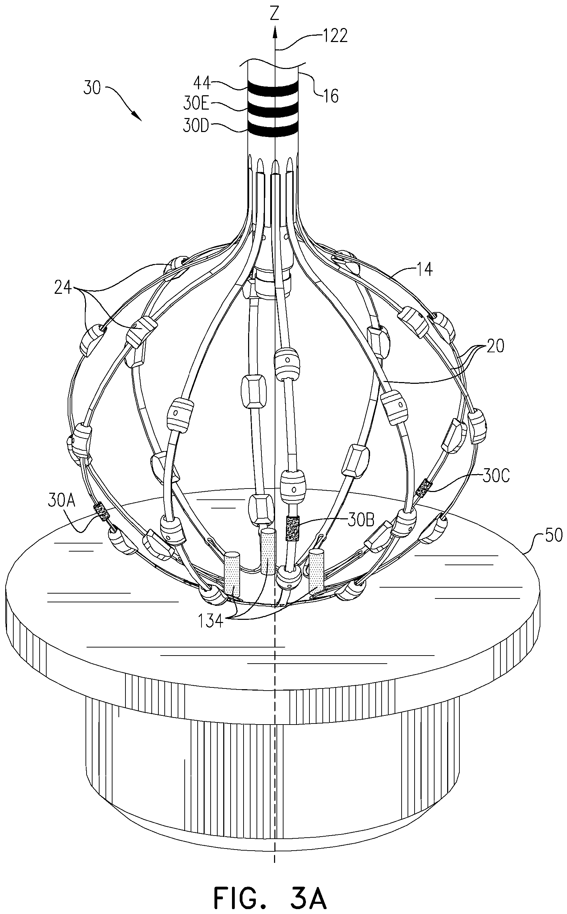

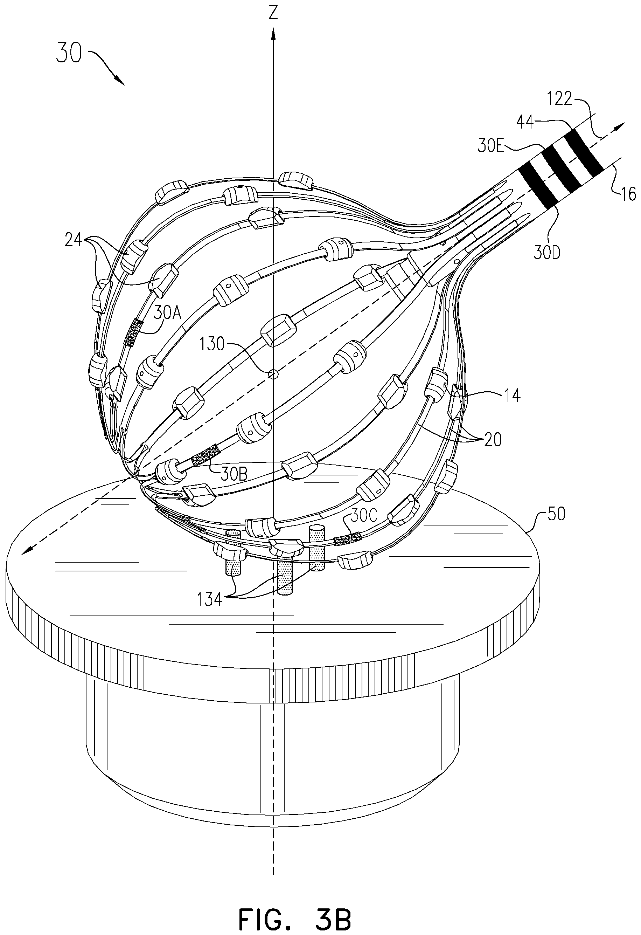

Reference is now made to Figs. 1, 2, and 3A and 3B which are respectively a schematic block diagram of a calibration system 10, schematic figures of the system, and schematic figures of a catheter distal end assembly 14 used in the calibration system, according to an example of the present disclosure. Distal end assembly 14 is attached to a shaft 16 of a catheter 18, and in Fig. 2 and Figs. 3A and 3B, distal end 14 is shown as a basket, having a plurality of generally similar splines 20 upon which are mounted generally similar electrodes 24. (In Fig. 1 the distal end assembly is shown schematically as a circle.) However, other distal end assemblies may be in the form of a balloon, so that distal end assembly 14 may also be referred to herein as basket 14 or balloon 14, and catheter 18 is also referred to as basket-type catheter 18.

System 10 comprises a jig 22, and two views of the jig are shown in Fig. 2. The views are drawn on xyz Cartesian axes, described further below. Jig 22 holds distal end assembly 14 and is operable to selectively deflect, twist and/or press distal end assembly 14 in a defined manner. During operation of the calibration system, a 3D strain gauge senses the applied strain due to the selective maneuvering of the distal end assembly and a location pad 26 is used to enable measurement of locations and orientations of sensors 30A, 30B, 30C, mounted on a distal section of distal end assembly 14, and of sensors 30D and 30E, mounted on the distal end of shaft 16. Sensors 30A, 30B, 30C, 30D, and 30E are also referred to herein as sensors 30, and each sensor generates respective signals indicative of the location and orientation of the sensor.

Sensors 30 are typically single axis sensors (SASs), but in some examples at least some of sensors 30 may comprise double axis sensors (DASs) and/or triple axis sensors (TASs). Sensors 30, together with location pad 26, are used for magnetic based position sensing, and details of magnetic based position sensing technology, which uses radiating alternating magnetic fields, are described in U.S. Patent Nos. 5,5391,199; 5,443,489; 5,558,091; 6,172,499; 6,239,724; 6,332,089; 6,484,118; 6,618,612; 6,690,963; 6,788,967; and 6,892,091.

Location pad 26 comprises a plurality of generally similar magnetic coils 28 each configured for generating a magnetic field, and in the present example there are three coils 28, two of which are visible in Fig. 2. The magnetic field from coils 28 induces a signal in sensors 30, and the signals from the sensors are used, as described below, to ascertain the position of each of sensors 30.

In some examples, a magnetic coil 44 configured for generating a magnetic field is located on the distal end of shaft 16, and sensors 30A, 30B, and 30C are configured to sense their location and orientation relative to coil 44, in response to a signal induced in the sensors due to the presence of the generated magnetic field from coil 44. In addition, signals may be induced on each of sensors 30A, 30B, 30C, 30D and 30E based on magnetic fields generated from coils 28 on the location pad. Each of the coils may be configured to generate a magnetic field in a dedicated AC frequency so that the signals induced on sensors 30A, 30B, 30C, 30D and 30E from the different generating coils may be differentiated.

A processor 34 transmits data for operating jig 22, as well as driving signals for the location pad. The processor receives force related data from the 3D strain gauge 36 in the jig, and in a disclosed example the strain gauge is also configured to provide torque related data to the processor. In an alternative example a separate torque-gauge 40, described further below, provides torque data to the processor. A relationship between strain and force (and/or torque) may be stored in memory associated with processor 34.

The processor also receives location and orientation data of each of sensors 30 via the position signals of the respective sensors, and the processor forms a correspondence between the two sets of data, i.e., between the force and torque data and the deflection of distal end assembly based on sensed location and orientation data. The correspondence may be used to find the force and torque on the distal end assembly of a basket-type catheter for different shaped deflections of the distal end assembly, for the same type of catheters used in a medical procedure. As stated above, strain gauge 36 provides force related data to processor 34.

Jig 22 comprises three actuators, which are controlled by processor 34, and which are used to selectively twist and deflect the distal end assembly. By way of example, in the present disclosure the actuators are assumed to comprise motors, but those having ordinary skill in the art will be aware of other types of actuators, such as linear translators or cables, and all such actuators are assumed to be comprised within the scope of the present disclosure.

A first actuator 38, also herein termed motor 38, is mounted on a fixed base 42 of the jig, and drives a cam 46. An inset 48 illustrates motor 38 and cam 46.

As is described below, operation of the motor causes cam 46 to move a platform 50, which supports distal end assembly 14, linearly. As stated above, views of jig 22 have been drawn on an orthogonal set of Cartesian xyz axes, and in the present description the x and the y axes are parallel to the edges of fixed base 42, and lie on the upper side of the fixed base, and the z axis is normal to the fixed base and passes through the center of platform 50, which also lies in an xy plane. In the description xy planes are also assumed to be horizontal planes, and the direction of z-axis is assumed to correspond to a vertical direction.

Cam 46 mates with pad 58 mounted on a moveable base 54, via a contact 58 embedded in the upper side of base 54, and the moveable base operates as a class 1 lever, and is also herein termed lever 54. Embedded in the underside of base 54 are two supports 62, which contact fixed base 42, and which act as a fulcrum for base 54 when the base operates as a lever. Thus, rotation of cam 46 by motor 38 provides a lever-effort to a section 66 of base 54, surrounding pad 58, pushing the section to move approximately linearly in a vertical direction.

As section 66 is moved linearly, a section 70 of base 54, on the opposite side of supports 62 to section 66, also moves approximately linearly and vertically, in the opposite direction to section 66, because base 54 acts as a lever. Thus, when a lever-effort is applied to section 66, a corresponding lever-load is produced on section 70. Section 70 mates with a strain gauge support bar 74, which in turn moves vertically, parallel to the z-axis, by being constrained to travel in vertical grooves 78, 82 in respective frame-supports 86, 90. Frame-supports 86, 90 are fixedly connected to fixed base 42 at its upper side.

Strain gauge support bar 74 supports strain gauge 36, which is connected on an upper surface of the cell to platform 50. The connection is configured so that strain gauge 36 provides force-signals corresponding to the vector force acting on platform 50. When implemented to measure torque, strain gauge 36 also provides torque-signals corresponding to the torque around the z-axis acting on the platform.

A second actuator 94, also herein termed motor 94, is mounted on frame-support 86 of the jig, and on operation is configured to rotate a frame 98 about an axis 102, parallel to the x-axis, passing through frame-supports 86 and 90. Since frame-supports 86 and 90 are fixed to base 42, axis 102 does not move with respect to the base. As is explained further below, rotation of frame 98 rotates distal end assembly 14 about axis of rotation 102, and the axis is configured to pass approximately through the center of the distal end assembly. Motor 94 uses a gear train 106 to rotate frame 98. As is illustrated, weights 110 are attached to frame 98, the weights being selected so that the frame is "balanced" around axis 102.

A third actuator 114, also herein termed motor 114, is mounted on frame 98. Also mounted on frame 98 is a catheter clamping assembly 118 that acts as a catheter support and that is configured to grip shaft 16 of catheter 18, and to rotate the shaft about an axis 122 defined by, and congruent with, shaft 16. In a disclosed example, a torque-gauge 40 is connected to assembly 118, and is configured to measure torque of shaft 16 about axis 122 when the shaft is rotated by actuator 114. Axis 122 is orthogonal to axis 102. Actuator 114 is coupled to clamping assembly 118 by a gear train 126, so that operation of actuator 114 causes shaft 16 of catheter 18 to rotate about the shaft, i.e., about axis 122.

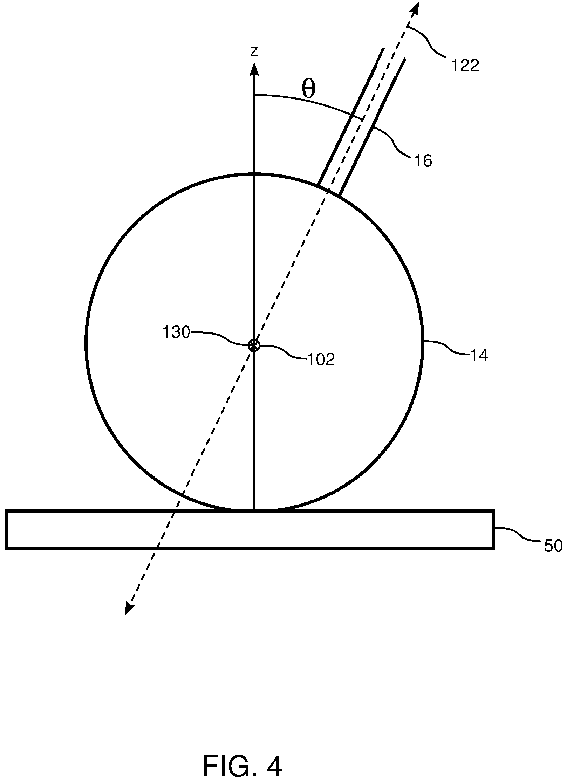

Reference is now made to Fig. 4, which is a schematic figure illustrating the relation between the z-axis, axis 102, and axis 122, according to an example of the present disclosure. In Fig. 4 distal end assembly 14 has been drawn schematically as a circle. In the figure, the z-axis and axis 122 lie in the plane of the paper, and axis 102 is orthogonal to the plane of the paper. In a disclosed example illustrated in Fig. 4, all three axes, the z-axis, axis 102, and axis 122 intersect at a common point 130. Jig 22 is configured so that common point 30 is approximately at the center of distal end assembly 14.

Returning to Figs. 1, 2, and 3A and 3B, it will be understood that when shaft 16 is gripped by clamping assembly 118, the jig may be considered to be in a "null" position wherein frame 98 is adjusted so that shaft 16 aligns with the z-axis. This is illustrated in Fig. 3A. Rotation of frame 98 about axis 102, from the null position, rotates distal end assembly 14 about axis 102, so that shaft 16 no longer aligns with the z-axis. A typical rotation is illustrated in Fig. 3B.

Operation of the Calibration System

In the description below, torque is assumed to be measured by strain gauge 36. Those having ordinary skill in the art will be able to adapt the description, mutatis mutandis, if torque is measured using torque-gauge 40. During operation of system 10, distal end assembly 14 may be translated and/or compressed along the z-axis, and may be rotated about axis 102 and axis 122. In order to maintain distal end assembly 14 in a preset location, wherein in a disclosed example the center of the distal end assembly is approximately at common point 130, platform 50 is configured to have a plurality of pillars 134 protruding from the platform. In the illustrated example there are three pillars 134 close to the center of platform 50, but other examples may have other numbers for the plurality, and/or other positions for the pillars.

When distal end assembly 14 is formed as a basket with splines 20, pillars 134 may be configured to interlock with the splines, as is illustrated in Fig. 3. Such interlocking prevents distal end assembly 14 from moving laterally, while still allowing the distal end assembly to be rotated, twisted, and/or compressed by the actions of actuators 38, 94, and 114. When distal end assembly 14 is formed as a balloon, pillars 134 may be moved further from the center of platform 50, so as to grip the balloon while still permitting rotation, twisting and/or compression of the balloon under the actions of the actuators.

As shown schematically in Fig. 1, during operation of system 10, processor 34 provides jig settings to jig 22, comprising settings for actuators 38, 94, and 114. Processor 34 also provides driving signals to coils 28 of location pad 26, and to coil 44. Processor 34 receives location and orientation signals from sensors 30 generated in response to their received magnetic radiations.

The processor receives force signals from strain gauge 36, indicative of the magnitude and the direction, in three dimensions (3D), of the force from distal end assembly 14 on the strain gauge. Strain gauge 36 also provides the processor with signals indicative of the torque about the z-axis on the strain gauge, that is generated by distal end assembly 14. Thus, the processor is able to determine the force and the torque exerted on pillars 134 of platform 50 due to twisting of the distal end assembly.

Actuator 114 twists shaft 16 about axis 122, and the splines of the distal end assembly apply a torque on pillars 134, around the z-axis. This is the torque that is measured. The torque around the z-axis is a projection of the torque around axis 122, and the two torques are related by equation (1):

$$T_z = \cos \theta \cdot T_s \qquad (1)$$

where $T_Z$ is the torque about the z-axis,

$T_S$ is the torque about catheter shaft 16, i.e. axis 122, and
$\Theta$ is the angle between the z-axis and the catheter shaft.
Angle $\Theta$ is illustrated in Fig. 4.

Equation (1) may be rearranged to equation (2):

$$T_s = \frac{T_z}{\cos \theta} \qquad (2)$$

Using the position data received from sensors 30, and the force and torque data received from strain gauge 36, and using equation (2), processor 34 is able to develop a correspondence between, on the one hand the positions of sensors 30, and on the other hand the 3D force on distal end assembly 14 and the torque around catheter shaft 16. The correspondence may be generated from a lookup table of the raw data received when system 10 is operated. Alternatively, the correspondence may be generated by fitting the raw data to a model applied to the distal end assembly, so as to find parameters of the model. In some examples splines 20 are elastic, and the model is assumed to be an elastic model. The correspondence may then be used to find the force and the torque of catheters other than catheter 18, when such catheters are used in a procedure. During the procedure, if the value of the torque is at a preset value, above which there may be the possibility that the distal end assembly may untwist suddenly, a warning that the preset value has been attained may be issued.

**Examples**

Example 1. Apparatus for calibrating a catheter (18), comprising:

a base (42) defining a first axis (z) orthogonal to the base;
a frame (98), coupled to the base, and configured to rotate about a second axis (102) orthogonal to the first axis;
a catheter support (118), coupled to the frame and configured to retain the catheter and to rotate the catheter about a third axis (122) orthogonal to the second axis; and
a gauge (36), coupled to the base and to a distal end assembly (14) of the catheter, and configured to provide an indication of a force on the distal end assembly in response to translation of the distal end assembly along the first axis.

Example 2. The apparatus according to example 1, and comprising an actuator, coupled to the frame, configured to rotate the frame about the second axis.
Example 3. The apparatus according to example 1, and comprising an actuator, coupled to the frame, configured to rotate the catheter about the third axis.
Example 4. The apparatus according to example 1, and comprising an actuator, coupled to the base, configured to provide the translation of the distal end assembly along the first axis.
Example 5. The apparatus according to example 4, and comprising a moveable base configured to act as a lever, and wherein the actuator is configured to exert an effort on the moveable base so as to exert the force on the distal end assembly.
Example 6. The apparatus according to example 5, wherein the moveable base is configured as a class 1 lever.
Example 7. The apparatus according to example 5, and comprising a cam coupled to the moveable base, and wherein the actuator is configured to rotate the cam so as exert the effort.
Example 8. The apparatus according to example 1, and comprising a platform, connecting the gauge to the distal end assembly, configured to support the distal end assembly.

Example 9. The apparatus according to example 8, comprising a plurality of pins, protruding from the platform, configured to retain the distal end assembly in a preset position on the platform.

Example 10. The apparatus according to example 1, and comprising a location pad, positioned in proximity to the base, configured to transmit electromagnetic radiation to the catheter and the distal end assembly, and wherein the electromagnetic radiation is configured to generate a signal in at least one sensor connected to at least one of the catheter and the distal end assembly, wherein the signal is indicative of a position of the at least one sensor.

Example 11. The apparatus according to example 1, wherein the gauge is configured to provide a further indication of a torque on the distal end assembly around the first axis in response to rotation of the catheter about the third axis.

Example 12. The apparatus according to example 11, and comprising a processor, configured to calculate an angle between the first axis and the third axis, and to calculate a further torque on the distal end assembly around the third axis in response to the angle and the indication of the torque around the first axis.

Example 13. The apparatus according to example 1, wherein the first axis, the second axis, and the third axis intersect at a common point.

Example 14. The apparatus according to example 1, and comprising a torque-gauge, coupled to the catheter support, configured to provide a further indication of a torque about the third axis in response to rotation of the catheter about the third axis.

Example 15. A method for calibrating a catheter (18), comprising:

providing a base (42) defining a first axis (z) orthogonal to the base;
coupling a frame (98) to the base, and configuring the frame to rotate about a second axis (102) orthogonal to the first axis;
coupling a catheter support (118) to the frame and configuring the catheter support to retain the catheter and to rotate the catheter about a third (122) axis orthogonal to the second axis;
coupling a gauge (36) to the base and to a distal end assembly (14) of the catheter; and
configuring the gauge to provide an indication of a force on the distal end assembly in response to translation of the distal end assembly along the first axis.

Example 16. The method according to example 15, and comprising coupling an actuator to the frame and configuring the actuator to rotate the frame about the second axis.

Example 17. The method according to example 15, and comprising coupling an actuator to the frame, and configuring the actuator to rotate the catheter about the third axis.

Example 18. The method according to example 15, and comprising coupling an actuator to the base, and configuring the actuator to provide the translation of the distal end assembly along the first axis.

Example 19. The method according to example 18, and comprising configuring a moveable base to act as a lever, and wherein the actuator is configured to exert an effort on the moveable base so as to exert the force on the distal end assembly.

Example 20. The method according to example 19, wherein the moveable base is configured as a class 1 lever.

Example 21. The method according to example 19, and comprising coupling a cam to the moveable base, and wherein the actuator is configured to rotate the cam so as exert the effort.

Example 22. The method according to example 15, and comprising connecting the gauge to the distal end assembly by a platform configured to support the distal end assembly.

Example 23. The method according to example 22, and comprising providing a plurality of pins, protruding from the platform, configured to retain the distal end assembly in a preset position on the platform.

Example 24. The method according to example 15, and comprising positioning a location pad in proximity to the base, and configuring the pad to transmit electromagnetic radiation to the catheter and the distal end assembly, and wherein the electromagnetic radiation is configured to generate a signal in at least one sensor connected to at least one of the catheter and the distal end assembly, wherein the signal is indicative of a position of the at least one sensor.

Example 25. The method according to example 15, wherein the gauge is configured to provide a further indication of a torque on the distal end assembly around the first axis in response to rotation of the catheter about the third axis.

Example 26. The method according to example 25, and comprising calculating an angle between the first axis and the third axis, and calculating a further torque on the distal end assembly around the third axis in response to the angle and the indication of the torque around the first axis.

Example 27. The method according to example 15, wherein the first axis, the second axis, and the third axis intersect at a common point.

Example 28. The method according to example 15, and comprising coupling a torque-gauge to the catheter support, and configuring the torque-gauge to provide a further indication of a torque about the third axis in response to rotation of the catheter about the third axis.

The examples described above are cited by way of example, and the present disclosure is not limited by what has been particularly shown and described hereinabove. Rather the scope of the disclosure includes both combinations and sub-combinations of the various features described hereinabove, as well as variations and modifications thereof which would occur to persons skilled in the art upon reading the foregoing description and which are not disclosed in the prior art.

**Claims**

1. Apparatus for calibrating a catheter (18), comprising:

    a base (42) defining a first axis orthogonal to the base;
    a frame (98), coupled to the base, and configured to rotate about a second axis (102) orthogonal to the first axis;
    a catheter support (118), coupled to the frame and configured to retain the catheter and to rotate the catheter about a third axis (122) orthogonal to the second axis; and
    a gauge (36), coupled to the base and to a distal end assembly (14) of the catheter, and configured to provide an indication of a force on the distal end assembly in response to translation of the distal end assembly along the first axis.

2. The apparatus according to claim 1, further comprising a first actuator, coupled to the frame, configured to rotate the frame about the second axis.

3. The apparatus according to claim 1, further comprising a second actuator, coupled to the frame, configured to rotate the catheter about the third axis.

4. The apparatus according to claim 1, further comprising a third actuator, coupled to the base, configured to provide the translation of the distal end assembly along the first axis, and optionally further comprising:

    a moveable base, wherein the moveable base is configured as a class 1 lever; and
    a cam coupled to the moveable base, and wherein the third actuator is configured to rotate the cam so as exert the effort.

5. The apparatus according to claim 1, further comprising:

    a platform, connecting the gauge to the distal end assembly, configured to support the distal end assembly; and
    a plurality of pins, protruding from the platform, configured to retain the distal end assembly in a preset position on the platform.

6. The apparatus according to claim 1, further comprising a location pad, positioned in proximity to the base, configured to transmit electromagnetic radiation to the catheter and the distal end assembly, and wherein the electromagnetic radiation is configured to generate a signal in at least one sensor connected to at least one of the catheter and the distal end assembly, wherein the signal is indicative of a position of the at least one sensor.

7. The apparatus according to claim 1, wherein the gauge is configured to provide a further indication of a torque on the distal end assembly around the first axis in response to rotation of the catheter about the third axis, and optionally further comprising a processor, configured to calculate an angle between the first axis and the third axis, and to calculate a further torque on the distal end assembly around the third axis in response to the angle and the indication of the torque around the first axis.

8. The apparatus according to claim 1, and comprising a torque-gauge, coupled to the catheter support, configured to provide a further indication of a torque about the third axis in response to rotation of the catheter about the third axis.

9. A method for calibrating a catheter (18), comprising:

    providing a base (42) defining a first axis orthogonal to the base;
    coupling a frame (98) to the base, and configuring the frame to rotate about a second axis (102) orthogonal to the first axis;
    coupling a catheter support (118) to the frame and configuring the catheter support to retain the catheter and to rotate the catheter about a third (122) axis orthogonal to the second axis;

coupling a gauge (36) to the base and to a distal end assembly (14) of the catheter; and
configuring the gauge to provide an indication of a force on the distal end assembly in response to translation of the distal end assembly along the first axis.

10. The method according to claim 9, further comprising coupling a first actuator to the frame and configuring the first actuator to rotate the frame about the second axis.

11. The method according to claim 9, further comprising coupling a second actuator to the frame, and configuring the second actuator to rotate the catheter about the third axis.

12. The method according to claim 9, further comprising coupling a third actuator to the base, and configuring the third actuator to provide the translation of the distal end assembly along the first axis, and optionally comprising a moveable base configured as a class 1 lever and coupling a cam to the moveable base, and wherein the third actuator is configured to rotate the cam so as exert the effort.

13. The method according to claim 9, further comprising:

connecting the gauge to the distal end assembly by a platform configured to support the distal end assembly; and
providing a plurality of pins, protruding from the platform, configured to retain the distal end assembly in a preset position on the platform.

14. The method according to claim 9, further comprising positioning a location pad in proximity to the base, and configuring the pad to transmit electromagnetic radiation to the catheter and the distal end assembly, and wherein the electromagnetic radiation is configured to generate a signal in at least one sensor connected to at least one of the catheter and the distal end assembly, wherein the signal is indicative of a position of the at least one sensor.

15. The method according to claim 9, wherein the gauge is configured to provide a further indication of a torque on the distal end assembly around the first axis in response to rotation of the catheter about the third axis, and optionally further comprising calculating an angle between the first axis and the third axis, and calculating a further torque on the distal end assembly around the third axis in response to the angle and the indication of the torque around the first axis.

16. The method according to claim 9, further comprising coupling a torque-gauge to the catheter support, and configuring the torque-gauge to provide a further indication of a torque about the third axis in response to rotation of the catheter about the third axis.

FIG. 1

9

ALTERNATIVE VIEWS
OF JIG22

PROCESSOR

FIG. 2

EP 4 570 212 A1

FIG. 3A

FIG. 3B

FIG. 4

Europäisches
Patentamt

European
Patent Office

Office européen
des brevets

## EUROPEAN SEARCH REPORT

Application Number

EP 24 22 0033

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/032152 A1 (GOVARI ASSAF [IL] ET AL) 30 January 2014 (2014-01-30) | 1-5,7, 9-13,15 | INV. A61B90/00 |
| Y | * paragraphs [0033], [0038] * <br> * figure 4 * | 6,8,14, 16 | A61B5/1495 |
| | ----- | | ADD. |
| Y | US 2019/353471 A1 (RACHELI NOAM [IL] ET AL) 21 November 2019 (2019-11-21) <br> * paragraphs [0047], [0048] * | 6,14 | A61B5/00 A61B18/00 |
| | ----- | | |
| Y | WO 2014/191871 A1 (KONINKL PHILIPS NV [NL]) 4 December 2014 (2014-12-04) <br> * page 12, line 1 - line 9 * | 8,16 | |
| | ----- | | |
| A | US 2011/153252 A1 (GOVARI ASSAF [IL] ET AL) 23 June 2011 (2011-06-23) <br> * paragraphs [0019] - [0039] * <br> * figures 1-3 * | 1-16 | |
| | ----- | | |

TECHNICAL FIELDS
SEARCHED      (IPC)

A61B

The present search report has been drawn up for all claims

| Place of search | Date of completion of the search | Examiner |
|---|---|---|
| The Hague | 2 May 2025 | Milles, Julien |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

                                                            
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 22 0033

This annex lists the patent family members relating to the patent documents cited in the above-mentioned European search report.
The members are as contained in the European Patent Office EDP file on
The European Patent Office is in no way liable for these particulars which are merely given for the purpose of information.

02-05-2025

| Patent document cited in search report | Publication date | Patent family member(s) | | Publication date |
|---|---|---|---|---|
| US 2014032152 A1 | 30-01-2014 | AU | 2010241467 A1 | 07-07-2011 |
| | | CA | 2722997 A1 | 23-06-2011 |
| | | CN | 102160820 A | 24-08-2011 |
| | | DK | 2338411 T3 | 06-01-2014 |
| | | EP | 2338411 A1 | 29-06-2011 |
| | | ES | 2448366 T3 | 13-03-2014 |
| | | IL | 209449 A | 30-09-2014 |
| | | JP | 5722023 B2 | 20-05-2015 |
| | | JP | 2011131059 A | 07-07-2011 |
| | | RU | 2010152701 A | 27-06-2012 |
| | | US | 2011153253 A1 | 23-06-2011 |
| | | US | 2014032152 A1 | 30-01-2014 |
| US 2019353471 A1 | 21-11-2019 | AU | 2019203343 A1 | 05-12-2019 |
| | | CA | 3043185 A1 | 15-11-2019 |
| | | CN | 110478031 A | 22-11-2019 |
| | | EP | 3569145 A1 | 20-11-2019 |
| | | IL | 266590 A | 29-08-2019 |
| | | JP | 7286407 B2 | 05-06-2023 |
| | | JP | 2019202134 A | 28-11-2019 |
| | | US | 2019353471 A1 | 21-11-2019 |
| WO 2014191871 A1 | 04-12-2014 | NONE | | |
| US 2011153252 A1 | 23-06-2011 | AU | 2010241464 A1 | 07-07-2011 |
| | | CA | 2723002 A1 | 23-06-2011 |
| | | CN | 102119871 A | 13-07-2011 |
| | | DK | 2338412 T3 | 13-01-2014 |
| | | EP | 2338412 A1 | 29-06-2011 |
| | | ES | 2448365 T3 | 13-03-2014 |
| | | IL | 209450 A | 30-07-2015 |
| | | JP | 5851091 B2 | 03-02-2016 |
| | | JP | 2011131058 A | 07-07-2011 |
| | | RU | 2010152699 A | 27-06-2012 |
| | | US | 2011153252 A1 | 23-06-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B